# EUROPÄISCHE PATENTSCHRIFT

(11) **EP 0 699 054 B1**
(45) Veröffentlichungstag und Bekanntmachung des Hinweises auf die Patenterteilung: **02.01.1997**
(21) Anmeldenummer: 94914367.1
(22) Anmeldetag: 13.04.1994
(51) Int. Cl.: A61B 17/28

(54) **CHIRURGISCHES INSTRUMENT MIT ZWEI TRENNBAREN GRIFFBRANCHEN**
SURGICAL INSTRUMENT WITH TWO SEPARABLE GRIPPING ARMS
INSTRUMENT A USAGE CHIRURGICAL COMPORTANT DEUX POIGNEES SEPARABLES

(30) Priorität: 19.05.1993 DE 4316768
(43) Veröffentlichungstag der Anmeldung: 06.03.1996
(73) Patentinhaber: AESCULAP AG, D-78532 Tuttlingen (DE)
(72) Erfinder: WEISSHAUPT, Dieter, D-78194 Immendingen (DE)
(74) Vertreter: Hoeger, Stellrecht & Partner
(86) Internationale Anmeldenummer: EP9401149
(87) Internationale Veröffentlichungsnummer: WO9426180

(56) Entgegenhaltungen:
- US-A- 3 814 102
- US-A- 4 369 788

## Beschreibung

Die Erfindung betrifft ein Instrument für chirurgische Zwecke mit zwei relativ zueinander verschiebbaren Arbeitsteilen und mit zwei relativ zueinander verschwenkbaren Griffbranchen, von denen eine mit den einen und die andere mit dem anderen Arbeitsteil derart in Verbindung stehen, daß beim Schließen beziehungsweise Öffnen der Griffbranchen die beiden Arbeitsteile gegeneinander verschoben werden, wobei eine der beiden Griffbranchen eine Welle zur schwenkbaren Lagerung der anderen Griffbranche trägt, die ihrerseits mit einem Mitnehmer an dem einen Arbeitsteil angreift.

Derartige Instrumente finden Verwendung beispielsweise bei sogenannten Rohrschaftinstrumenten oder bei chirurgischen Stanzen, bei denen zwei parallel zueinander verschiebbare Arbeitsteile über die beiden Griffbranchen verschoben werden. Dazu sind die Griffbranchen, die im wesentlichen senkrecht von der Längsausdehnung der Arbeitsteile abstehen, schwenkbar miteinander verbunden, die Lagerung erfolgt dabei durch eine Lagerwelle an einer Griffbranche. Zwischen einer der beiden Griffbranchen und einem Arbeitsteil ist außerdem eine gelenkige Verbindung vorgesehen, so daß die Schwenkbewegung der Griffbranche in eine translatorische Bewegung des Arbeitsteils umgesetzt werden kann (US-A-3 814 102).

Es ist Aufgabe der Erfindung, ein derartiges Instrument so auszubilden, daß ein Zerlegen der Einzelteile, beispielsweise zum Zwecke der Reinigung, trotz dieses relativ komplizierten Aufbaus einfach vorgenommen werden kann.

Diese Aufgabe wird bei einem Instrument der eingangs beschriebenen Art erfindungsgemäß dadurch gelöst, daß die Welle Bereiche mit unterschiedlichem Durchmesser aufweist und derart verschiebbar ist, daß im Bereich der anderen Griffbranche teilweise ein Bereich größeren oder ein Bereich geringeren Durchmessers wirksam ist, daß die andere Griffbranche einen sich schlüssellochförmig erweiternden Einführschlitz mit einem schmaleren Einschubabschnitt und einem erweiterten Lagerabschnitt für die Welle aufweist, deren Breite im schmalen Einschubabschnitt zwischen den Durchmessern der beiden Bereiche der Welle liegt und im kreisförmig erweiterten Lagerabschnitt einen Durchmesser aufweist, der dem größeren Durchmesser der Welle entspricht.

Eine derartige Konstruktion ermöglicht es, eine sichere Lagerung der beiden Griffbranchen dadurch zu erreichen, daß die Welle mit dem Bereich größeren Durchmessers in den erweiterten Lagerabschnitt des Mitnehmers eingreift. In diesem Bereich wird das verschiebliche Arbeitsteil durch den Mitnehmer mitgenommen. Zur Lösung der Teile genügt es, die Lagerwelle so zu verschieben, daß der Bereich mit geringerem Durchmesser am erweiterten Lagerabschnitt des Einführschlitzes wirksam wird, dann kann die Griffbranche mit dem Einführschlitz an der Welle entlanggeführt werden, so daß dadurch der Mitnehmer aus seiner Wirkverbindung mit dem Arbeitsteil austritt. Sobald die Welle den Einführschlitz verlassen hat, ist eine vollständige Trennung der Griffbranche einerseits von der anderen Griffbranche und andererseits von dem mitgenommenen Arbeitsteil erfolgt, das dann einfach vom anderen Arbeitsteil durch weiteres Verschieben getrennt werden kann. Um diese Lösung und im umgekehrten Falle eine Festlegung der Griffbranche zu ermöglichen, genügt es, die Welle so zu verschieben, daß wahlweise die Bereiche mit größerem beziehungsweise kleinerem Durchmesser an der anderen Griffbranche wirksam werden.

Dabei ist es vorteilhaft, wenn die Welle unter Federbelastung in eine Position verschoben wird, in der der Bereich mit größerem Durchmesser an der auf der Welle gelagerten Griffbranche wirksam ist. Dies ist der normale Arbeitsbereich, in dem die Griffbranchen verschwenkbar aneinander gelagert sind. Nur gegen die Wirkung dieser Feder läßt sich die Welle in eine Position verschieben, in der eine Lösung der beiden Griffbranchen möglich ist. Beim Loslassen der Welle wird daher nach dem Zusammenfügen der beiden Griffbranchen die Welle automatisch in die Verriegelungsposition verschoben, in der ein Lösen nicht mehr möglich ist.

Der Mitnehmer kann beispielsweise in eine Ausnehmung des Arbeitsteils eintauchen, bei einer bevorzugten Ausführungsform ist jedoch vorgesehen, daß der Mitnehmer durch den Einschubteil des Einführschlitzes gebildet ist, der ein Mitnahmeglied am Arbeitsteil an gegenüberliegenden Seiten umgreift. Dabei ist es vorteilhaft, wenn das Mitnahmeglied ein parallel zur Lagerwelle verlaufender Bolzen mit einem kreisförmigen Querschnitt ist.

Weiterhin hat es sich dabei als günstig erwiesen, wenn der Einführschlitz an seinem Ende abgebogen ist, so daß über den gesamten Schwenkbereich der Griffbranchen jeweils eine im wesentlichen senkrecht zur Verschieberichtung des Arbeitsteils angeordnete Fläche des Einführschlitzes am Mitnahmeglied anliegt.

Die Lagerwelle kann beispielsweise längs ihres Umfanges Bereiche mit unterschiedlichem Durchmesser aufweisen und um ihre Längsachse drehbar sein. Beispielsweise ist ein Querschnitt vorstellbar, der ausgeht von einem kreisförmigen Querschnitt, wobei an gegenüberliegenden Seiten die Welle abgeflacht ist. Die Abflachung wird dabei so gewählt, daß in der einen Winkelstellung die Welle durch den Einschubabschnitt des Einführschlitzes hindurch paßt, in der anderen Winkelstellung jedoch nicht. Dadurch läßt sich einfach durch Verdrehung der Welle nach dem Einschieben dieser Griffbranche eine Verriegelung erreichen.

Bei einer anderen, bevorzugten Ausführungsform ist vorgesehen, daß die Welle axial verschieblich an der Griffbranche gelagert ist und daß die Bereiche unterschiedlichen Durchmessers in axialer Richtung der Welle nebeneinander angeordnet sind. Hier werden Bereiche unterschiedlichen Durchmessers der Welle durch axiale Verschiebung der Welle in den Bereich des Einführschlitzes verschoben.

Günstig ist es, wenn die Welle über die gelagerte Griffbranche mindestens über den axialen Verschiebeweg der Welle hervorsteht. Dadurch ist es möglich, durch Druck auf den vorstehenden Teil die axiale Verschiebung vorzunehmen, und zwar so weit, bis die Welle in Lösestellung verschoben ist.

Dabei kann weiterhin vorgesehen sein, daß die Welle im über die gelagerte Griffbranche vorstehenden Bereich einen Durchmesser aufweist, der größer ist als der Durchmesser des erweiterten Lagerabschnitts des Einführschlitzes. Einmal wird dadurch die Handhabung erleichtert, da der Bedienungsperson eine Druckfläche zur Verfügung steht, die auch bei einem sehr kleinen Durchmesser der Welle ausreichend groß sein kann, zum anderen kann dieser Bereich größeren Durchmessers als Anschlag verwendet werden, der die Einschubtiefe der Welle begrenzt.

Die nachfolgende Beschreibung bevorzugter Ausführungsformen der Erfindung dient im Zusammenhang mit der Zeichnung der näheren Erläuterung. Es zeigen:

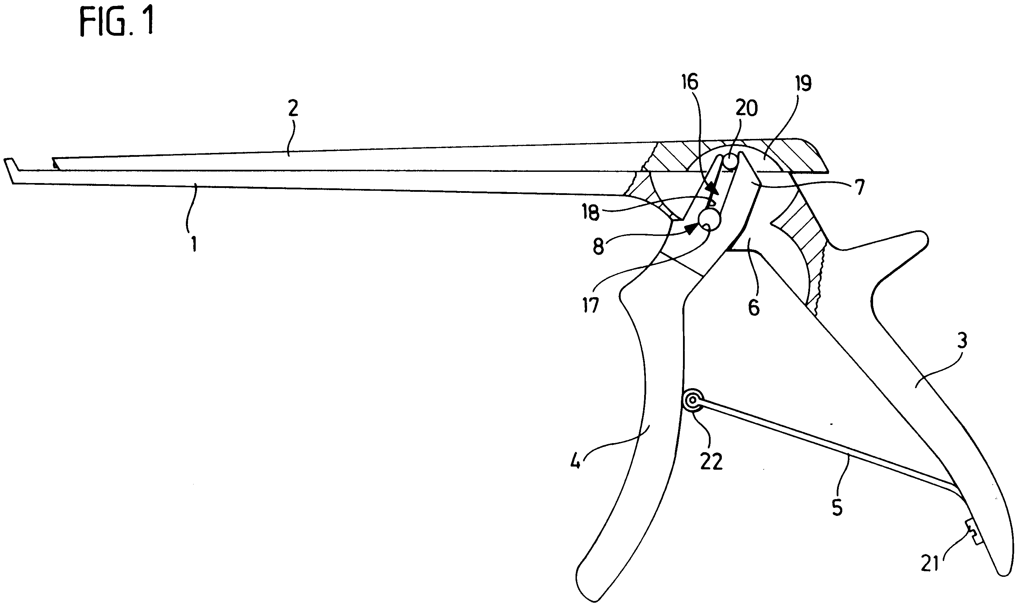

- Figur 1:: eine schematische Seitenansicht eines chirurgischen Instruments;
- Figur 2:: eine Ansicht des Instruments der Figur 1 im Schlußbereich im zerlegten Zustand;

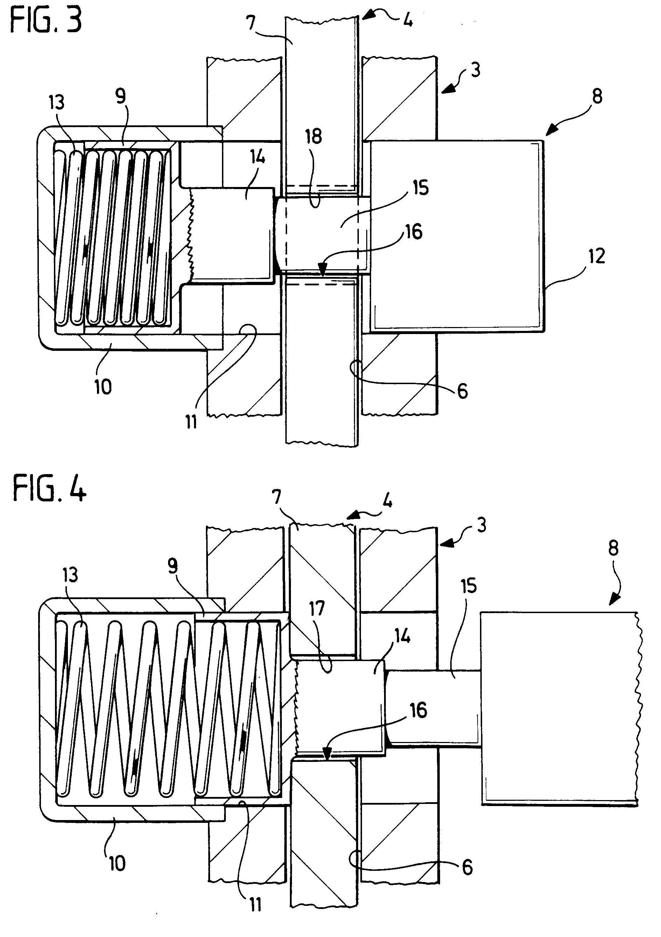

- Figur 3:: eine Längsschnittansicht des Instruments der Figur 1 im Lagerbereich der beiden Griffbranchen mit der Welle im unverriegelten Zustand und
- Figur 4:: eine Ansicht ähnlich Figur 3 mit der Lagerwelle im verriegelten Zustand.

Das in der Zeichnung dargestellte Instrument weist zwei längliche Arbeitsteile 1 und 2 auf, die durch eine in der Zeichnung nicht dargestellte Längsführung parallel zueinander verschieblich sind. Es kann sich beispielsweise um eine Gewebestanze handeln, wobei am freien Ende der beiden Arbeitsteile 1 und 2 stanzenartige Werkzeuge angeordnet sind, die beim Gegeneinanderschieben der beiden Arbeitsteile 1 und 2 dazwischenliegendes Gewebe erfassen und einen Teil daraus ausstanzen.

Das untere Arbeitsteil 1 ist einstückig mit einer Griffbranche 3 verbunden, an der um eine quer zur Längsrichtung der Arbeitsteile 1 und 2 verlaufende Drehachse eine zweite Griffbranche 4 schwenkbar gelagert ist. Zwischen beiden Griffbranchen ist eine Biegefeder 5 angeordnet, die die beiden Griffbranchen voneinander entfernt, so daß die beiden Griffbranchen 3 und 4 gegen eine Federkraft aufeinanderzu schwenkbar sind.

Zur Lagerung der Griffbranche 4 an der Griffbranche 3 weist die Griffbranche 3 einen parallel zur Längsrichtung der Arbeitsteile 1 und 2 verlaufenden, von oben nach unten durchgehenden Schlitz 6 auf, in den ein an der anderen Griffbranche 4 angeformter Lappen 7 eintaucht, dessen Breite im wesentlichen der Breite des Schlitzes 6 entspricht, so daß die Griffbranche 4 in diesem Schlitz 6 seitlich festgelegt ist.

Die Griffbranche 3 trägt im Bereich der Lagerung eine Welle 8, die länger ist als die Breite der Griffbranche 3 und somit an beiden Seiten über die Griffbranche 3 hervorsteht. Auf der einen Seite taucht die Welle 8 mit einem napfförmig ausgebildeten Ende 9 in einen Becher 10 ein, der in eine Stufenbohrung 11 in der Griffbranche 3 eingepreßt ist. Diese Stufenbohrung 11 durchsetzt die gesamte Griffbranche 3, sie weist einen erweiterten Bereich an einer Seite auf, dort ist der Becher 10 in die Bohrung eingepaßt. Der restliche Bereich der Stufenbohrung 11 mit geringerem Durchmesser entspricht in seinem Innendurchmesser dem Innendurchmesser des Bechers 10, der auch dem Außendurchmesser des napfförmig ausgebildeten Endes 9 und des gegenüberliegenden Endes 12 der Welle 8 entspricht. Dadurch ist die Welle 8 in dem Becher 10 und in der Stufenbohrung 11 in axialer Richtung frei verschieblich, in radialer Richtung jedoch festgelegt.

In dem Becher 10 ist eine von dem napfförmigen Ende 9 der Welle 8 teilweise umgebene Schraubenfeder 13 angeordnet, die sich am Boden des Bechers 10 und an der Stirnseite der Welle 9 abstützt und somit die Welle 8 mit einer aus dem Becher 10 heraustreibenden Kraft beaufschlagt.

Im Bereich zwischen dem napfförmigen Ende 9 und dem Ende 12 der Welle weist diese zwei nebeneinanderliegende Bereiche mit geringerem Durchmesser auf, nämlich einen an das Ende 9 anschließenden Bereich 14 und einen an das Ende 12 anschließenden Bereich 15, dessen Durchmesser noch geringer ist als der des Bereichs 14. In axialer Richtung erstrecken sich die Bereiche 14 und 15 jeweils über eine Strecke, die größer ist als die Breite des Schlitzes 6.

Im Lappen 7 der Griffbranche 4 ist ein sich schlüssellochförmig erweiternder Einführschlitz 16 angeordnet, der einen im Querschnitt kreisförmigen Lagerabschnitt 17 und einen radial aus diesem austretenden Einschubabschnitt 18 aufweist. Dieser ist im wesentlichen geradlinig ausgebildet, nur am oberen Ende des Lappens weist der Einschubabschnitt 18 eine geringfügige Krümmung auf. Der Durchmesser des Lagerabschnitts 17 entspricht dem Durchmesser des Bereichs 14 auf der Welle 8, die Breite des Einschubabschnitts 18 entspricht dem Durchmesser des Bereichs 15 der Welle 8.

Im oberen Arbeitsteil 2 ist ausgerichtet mit dem Schlitz 6 in der Griffbranche 3 ebenfalls ein Schlitz 19 eingearbeitet, durch den ein parallel zur Welle 8 verlaufender, im Querschnitt kreisförmiger Bolzen 20 hindurchtritt.

Ein Zusammenbau der beschriebenen Teile des Instruments wird damit begonnen, daß die beiden Arbeitsteile 1 und 2 längs ihrer relativen Führung parallel zueinander so verschoben werden, daß der Bolzen 20 etwa oberhalb der Stufenbohrung 11 steht. Anschließend wird die vordere Griffbranche 4 mit ihrem Lappen 7 von unten her in den Schlitz 6 eingeführt, bis das Ende des Einschubabschnitts 18 an der Welle 8 anstößt. Durch die Wirkung der Schraubenfeder 13 befindet sich der Bereich 14 mit dem größeren Durchmesser am Ende des Einschubabschnitts 18, so daß eine weitere Verschiebung des Lappens 7 nicht möglich ist. Um ein weiteres Einschieben des Lappens 7 zu ermöglichen, wird die Welle 8 durch Druck auf deren Ende 12 gegen die Wirkung der Schraubenfeder 13 in den Becher 10 hineingeschoben, bis der Bereich 15 mit dem geringeren Durchmesser den Schlitz 6 überbrückt. Da der Durchmesser des Bereichs 15 gleich oder geringfügig kleiner ist als die Breite des Einschubabschnitts 18, kann nunmehr der Lappen 7 so weit in den Schlitz 6 eingeschoben werden, bis das Ende des Einschubabschnitts 18 den Bolzen 20 beidseitig umgreift und bis die Welle 8 den Lagerabschnitt 17 des Einführschlitzes 16 erreicht. Läßt man in dieser Position die Welle 8 los, so wird sie unter der Wirkung der Schraubenfeder 13 axial verschoben, und der Bereich 14 mit dem größeren Durchmesser tritt in den Lagerabschnitt 17 ein, dessen Durchmesser im wesentlichen dem des Bereichs 14 entspricht. Dadurch ergibt sich eine einwandfreie Lagerung der Griffbranche 4 an der Griffbranche 3. Die Biegefeder 5, die beispielsweise mit einer Schraube 21 an der Griffbranche 3 festgelegt ist, kann in eine lösbare Halterung 22 an der Griffbranche 4 eingehängt werden oder sie liegt über eine Rolle an der Griffbranche 4 an, damit ist das Instrument betriebsfertig. Beim Verschwenken der Griffbranche 4 gegenüber der Griffbranche 3 wird der Bolzen 20 längs der Verschiebebahn des Arbeitsteils 2 mitgenommen und kann dabei im Einschubabschnitt 18 des Einführschlitzes 16 in Längsrichtung verschoben werden, dieser Bolzen 20 bildet somit ein Mitnahmeglied, das mit dem einen Mitnehmer bildenden Einführschlitz 16 zusammenwirkt. Dabei ist wesentlich, daß die translatorische Bewegung des Arbeitsteils 1 unbehindert erfolgen kann, der Bolzen 20 kann sich also längs einer Gerade bewegen, während der Einführschlitz 16 um die durch die Welle 8 bestimmte Drehachse verschwenkt wird.

Ein Lösen kann in umgekehrter Richtung dadurch erfolgen, daß die Welle 8 entgegen der Schraubenfeder 13 axial so weit verschoben wird, bis der Bereich 15 mit geringerem Außendurchmesser den Schlitz 6 überbrückt. Es ist dann ohne weiteres möglich, gegebenenfalls nach Lösen der Halterung 22, die Griffbranche 4 längs des Einführschlitzes 16 aus dem Schlitz 6 herauszuziehen. Nach der Entfernung der Griffbranche 4 läßt sich auch das obere Arbeitsteil 2 relativ zum unteren Arbeitsteil 1 nach hinten vollständig herausschieben.

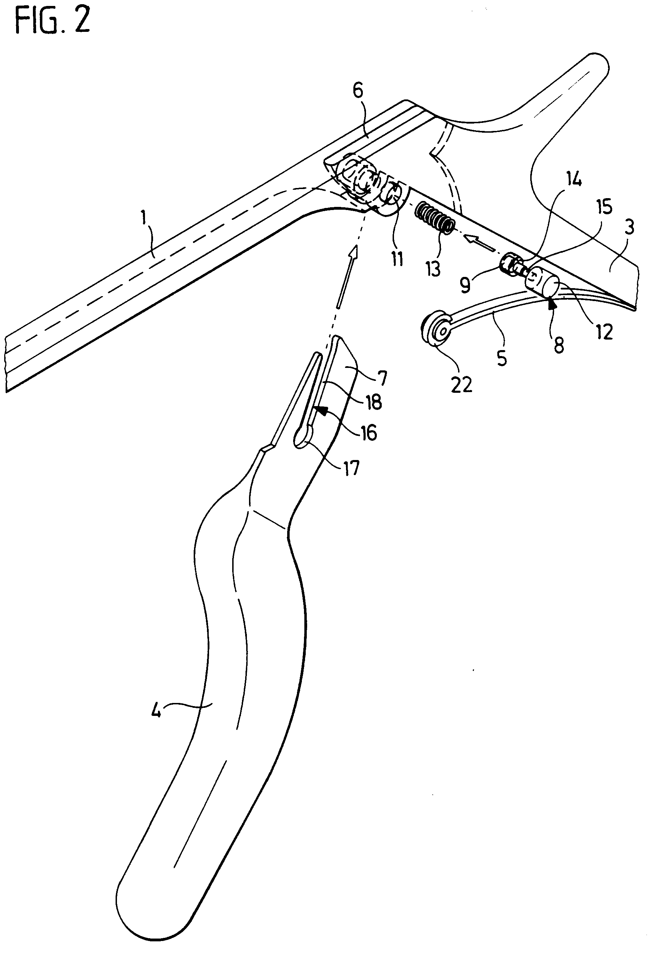

Wie aus der Darstellung der Figur 2 ersichtlich, ist es grundsätzlich auch möglich, die Welle 8 zu Reinigungszwecken zu entnehmen, dies gelingt nach dem Herausziehen der vorderen Griffbranche 4 einfach dadurch, daß die Welle 8 durch die Stufenbohrung 1 herausgezogen wird, auch die Schraubenfeder 13 kann nach Entnahme der Welle 8 in gleicher Weise aus dem Becher 10 herausgezogen werden.

Man erhält also auf diese Weise eine besonders einfache Lagerung, die schnell gelöst und hergestellt werden kann und bei der alle Einzelteile in einfachster Weise zusammensetzbar und zu Reinigungszwecken herausnehmbar sind.

## Patentansprüche

1. Instrument für chirurgische Zwecke mit zwei relativ zueinander verschiebbaren Arbeitsteilen (1, 2) und mit zwei relativ zueinander verschwenkbaren Griffbranchen (3, 4), von denen eine mit dem einen und die andere mit dem anderen Arbeitsteil derart in Verbindung stehen, daß beim Schließen beziehungsweise Öffnen der Griffbranchen (3, 4) die beiden Arbeitsteile (1, 2) gegeneinander verschoben werden, wobei eine der beiden Griffbranchen (3) eine Welle (8) zur schwenkbaren Lagerung der anderen Griffbranche (4) trägt, die ihrerseits mit einem Mitnehmer an dem einen Arbeitsteil (2) angreift,
dadurch gekennzeichnet, daß die Welle (8) Bereiche (14, 15) mit unterschiedlichem Durchmesser aufweist und derart verschiebbar ist, daß im Bereich der anderen Griffbranche (4) wahlweise ein Bereich (14) größeren oder ein Bereich (15) geringeren Durchmessers wirksam ist, daß die andere Griffbranche (4) einen sich schlüssellochförmig erweiternden Einführschlitz (16) mit einem schmaleren Einschubabschnitt (18) und einem erweiterten Lagerabschnitt (17) für die Welle (8) aufweist, deren Breite im schmaleren Einschubabschnitt (18) zwischen dem Durchmesser der beiden Bereiche (14, 15) der Welle (8) liegt und im kreisförmig erweiterten Lagerabschnitt (17) einen Durchmesser aufweist, der dem größeren Durchmesser (Bereich 14) der Welle (8) entspricht.

2. Instrument nach Anspruch 1, dadurch gekennzeichnet, daß die Welle (8) unter Federbelastung in eine Position verschoben wird, in der der Bereich (14) mit größerem Durchmesser an der auf der Welle (8) gelagerten Griffbranche (4) wirksam ist.

3. Instrument nach Anspruch 1 oder 2, dadurch gekennzeichnet, daß der Mitnehmer durch den Einschubabschnitt (18) des Einführschlitzes (16) gebildet ist, der ein Mitnahmeglied (20) im Arbeitsteil (2) an gegenüberliegenden Seiten umgreift.

4. Instrument nach Anspruch 3, dadurch gekennzeichnet, daß das Mitnahmeglied (20) ein parallel zur Welle (8) verlaufender Bolzen mit einem kreisförmigen Querschnitt ist.

5. Instrument nach einem der voranstehenden Ansprüche, dadurch gekennzeichnet, daß der Einführschlitz (16) an seinem Ende abgebogen ist.

6. Instrument nach einem der voranstehenden Ansprüche, dadurch gekennzeichnet, daß die Welle (8) axial verschieblich an der Griffbranche (3) gelagert ist und daß die Bereiche (14, 15) unterschiedlichen Durchmessers in axialer Richtung der Welle (8) nebeneinander angeordnet sind.

7. Instrument nach Anspruch 6, dadurch gekennzeichnet, daß die Welle (8) über die gelagerte Griffbranche (4) mindestens um den maximalen axialen Verschiebeweg der Welle (8) hervorsteht.

8. Instrument nach Anspruch 7, dadurch gekennzeichnet, daß die Welle (8) im über die gelagerte Griffbranche (4) vorstehenden Bereich einen Durchmesser aufweist, der größer ist als der Durchmesser des erweiterten Lagerabschnitts (17) des Einführschlitzes (16).

## Claims

1. An instrument for surgical use, comprising two working parts (1, 2) displaceable relative to one another and two gripping arms (3, 4) pivotable relative to one another, one of which is connected to one of the working parts and the other being connected to the other working part in such a manner that the two working parts (1, 2) are mutually displaced when the gripping arms (3, 4) are opened or closed, wherein one of the two gripping arms (3) carries a spindle (8) for pivotably mounting the other gripping arm (4) which for its part engages the one working part (2) by means of a carrier, characterised in that the spindle (8) has regions (14, 15) of different diameters and is displaceable in such a manner that, in the region of the other gripping arm (4), either a region (14) of larger diameter or a region (15) of smaller diameter is operative, in that the other gripping arm (4) has an insertion slot (16) widening in the manner of a keyhole and having a narrower slide-in portion (18) and a widened mounting portion (17) for the spindle (8), the width of which in the narrower slide-in portion (18) lies between the diameter of the two regions (14, 15) of the spindle (8) and, in the mounting portion (17) widened in the shape of a circle, has a diameter corresponding to the larger diameter (region 14) of the spindle (8).

2. An instrument according to claim 1, characterised in that the spindle (8) is displaced under spring load into a position in which the region (14) of larger diameter is operative on the gripping arm (4) mounted on the spindle (8).

3. An instrument according to claim 1 or 2, characterised in that the carrier is formed by the slide-in portion (18) of the insertion slot (16) and engages round opposite sides of a carrier member (20) in the working part (2).

4. An instrument according to claim 3, characterised in that the carrier member (20) is a pin extending parallel to the spindle (8) and having a circular cross-section.

5. An instrument according to any one of the preceding claims, characterised in that the end of the insertion slot (16) is angled.

6. An instrument according to any one of the preceding claims, characterised in that the spindle (8) is axially displaceably mounted on the gripping arm (3) and in that the regions (14, 15) of different diameters are arranged side by side in the axial direction of the spindle (8).

7. An instrument according to claim 6, characterised in that the spindle (8) projects beyond the mounted gripping arm (4) by at least the maximum axial displacement path of the spindle (8).

8. An instrument according to claim 7, characterised in that, in the region projecting beyond the mounted gripping arm (4), the spindle (8) has a diameter which is greater than the diameter of the widened mounting portion (17) of the insertion slot (16).

## Revendications

1. Instrument à usage chirurgical possédant deux parties travaillantes (1, 2) qui peuvent coulisser l'une par rapport à l'autre et deux branches de poignée (3, 4) qu'on peut faire pivoter l'une par rapport à l'autre, dont l'une est reliée à une partie travaillante et l'autre à l'autre partie travaillante, de telle manière que, lors de l'ouverture ou de la fermeture des branches de poignée (3, 4), les deux parties travaillantes (1, 2) se déplacent en translation l'une par rapport à l'autre, l'une (3) des deux branches de poignée portant un axe (8) pour l'articulation de l'autre branche de poignée (4), laquelle attaque à son tour l'une (2) des parties travaillantes au moyen d'un taquet,
caractérisé en ce que l'axe (8) présente des régions (14, 15) ayant différents diamètres et peut être déplacé en translation de telle manière que, dans la région de l'autre branche de poignée (4), ce soit tantôt une région (14) de plus grand diamètre, tantôt une région (15) de plus petit diamètre qui entre en jeu, en ce que l'autre branche de poignée (4) présente une fente d'introduction (16) qui s'élargit en forme de trou de serrure et qui comprend un segment d'emmanchement (18) plus étroit et un segment d'articulation (17) élargi destiné à recevoir l'axe (8) et dont la largeur est comprise entre les diamètres des deux régions (14, 15) de l'axe (8) dans le segment étroit d'emmanchement (18) et présente un diamètre qui correspond au plus grand diamètre (région 14) de l'axe (8) dans le segment d'articulation (17) élargi en forme de cercle.

2. Instrument selon la revendication 1, caractérisé en ce que l'axe (8) est repoussé, sous contrainte élastique, dans une position dans laquelle la région (14) de plus grand diamètre agit sur la branche (4) de la poignée qui est articulée sur l'axe (8).

3. Instrument selon la revendication 1 ou 2, caractérisé en ce que le taquet est formé par le segment d'engagement (18) de la fente d'introduction (16) qui encadre sur les deux côtés un organe d'entraînement (20) prévu dans la partie travaillante (2).

4. Instrument selon la revendication 3, caractérisé en ce que l'organe d'entraînement (20) est un tourillon à section transversale circulaire qui s'étend parallèlement à l'axe (8).

5. Instrument selon une des revendications précédentes, caractérisé en ce que la fente d'introduction (16) est incurvée à son extrémité.

6. Instrument selon une des revendications précédentes, caractérisé en ce que l'axe (8) est monté mobile en translation axiale sur la branche (3) de la poignée et en ce que les régions (14, 15) d'un diamètre différent sont juxtaposées dans la direction axiale de l'axe (8).

7. Instrument selon la revendication 6, caractérisé en ce que l'axe (8) déborde au-delà de la branche articulée (4) de la poignée d'une distance au moins égale à la course maximale de translation axiale de l'axe (8).

8. Instrument selon la revendication 7, caractérisé en ce que l'axe (8) présente, dans la région qui déborde au-delà de la branche articulée (4) de la poignée, un diamètre qui est plus grand que le diamètre du segment d'articulation élargi (17) de la fente d'introduction (16).
